# EUROPEAN PATENT APPLICATION

(11) **EP 3 770 593 A1**
(43) Date of publication of application: **27.01.2021**
(21) Application number: 19188711.6
(22) Date of filing: 26.07.2019
(51) Int. Cl.: G01N 27/28, G01N 27/48, G01N 33/00

(54) **USE OF A DEVICE COMPRISING A POROUS ELECTRODE AND AN ELECTRICALLY INSULATING POROUS LAYER TO REMOVE OXYGEN IN CONTACT WITH A WORKING ELECTRODE**

(71) Applicant: Université de Lorraine, 54000 Nancy (FR); Centre National de la Recherche Scientifique, 75016 Paris (FR)
(72) Inventor: ETIENNE, Mathieu, 54500 Vandoeuvre-lès-Nancy (FR); HERZOG, Grégoire, 54600 Villers-lès-Nancy (FR); NASIR, Tauqir, 54000 Nancy (FR); LE, Thi Xuan Huong, Englewood, OH 45322-2129 (US)
(74) Representative: Gevers & Orès

(57) **Abstract**

The present invention concerns the use of a device comprising a porous electrode and an electrically insulating porous layer to remove oxygen in contact with a working electrode. The present invention also concerns the use of said device in contact to said working electrode to detect and/or quantify an analyte in presence of oxygen.

## Description

The present invention concerns the use of a device comprising a porous electrode and an electrically insulating porous layer to remove oxygen in contact with a working electrode. The present invention also concerns the use of said device in contact to said working electrode to detect and/or quantify an analyte in presence of oxygen.

Electrochemical techniques offer the advantage of being label-free, rapid, simple and cost-efficient. Nevertheless, an intrinsic problem in electroanalytical detection is that the analytical current is the sum of all (possibly interfering) currents of the electroactive species present in solution, including oxygen from air. Moreover, oxygen can be competing during the detection of some species, e.g. low metal concentration, leading to a degradation of the analytical performances of the sensor.

Indeed, oxygen is soluble in water and is therefore likely to be found in aqueous solutions that are in contact or have been in contact with air.

However, some sensing reactions in aqueous media are hindered by the presence of dissolved oxygen. Thus the removal of dissolved oxygen plays a crucial role in these sensor applications, notably under ambient air. In particular, it is often necessary to detect a given analyte in aqueous medium at a small concentration. But this detection cannot be performed in presence of dissolved oxygen, the detection efficiency depending on the O₂ removal ability since oxygen induces a significant error in analyte detection, especially at very low analyte concentrations.

Therefore, in many cases, oxygen has to be removed from water as completely as possible.

Present methods of oxygen removal are physical or chemical deoxygenation. An easy and common physical method to eliminate dissolved oxygen is purging solution with nitrogen or argon.The main disadvantages of the physical methods is the high capital cost, for example in outdoor applications where compress nitrogen or argon needs to be carried by the user.

Chemical deoxygenation is based on chemicals reacting with oxygen. To avoid using of N₂ or Ar, ascorbic acid has been used as a reducing agent to remove interfering oxygen for the greenhouse gas nitrous oxide (N₂O) detection which is very sensible toward dioxygen. Other oxygen scavengers like sodium thiosulfate (Na₂S₂O₃), phosphines, also proved its efficient ability for this purpose. The sensing of H₂O₂ could be done under both air-saturated and O₂-saturated solution at polyaniline modified Pt electrode by adding Na₂S₂O₃ with concentration below 1 mM. For application in N₂O sensor, phosphines soluble in organic solvents showed a high sensitivity and long response time. An enzymatic oxygen scavenging system using different oxidase enzymes such as glucose, galactose or pyranose 2-oxidase as effective catalysts for O₂ reduction has been known recently. One of the main drawbacks with the chemical and enzymatic methods is the need of a continuous addition of reagent to the reactor.

Accordingly, it is an object of the present invention to provide devices, uses and processes that have high efficiency with a low energy and chemicals consumption.

Inventors have for the first time demonstrated that devices, uses and processes of the invention enable to only remove oxygen in an area confined to the surface of the sensor, quickly allowing an oxygen concentration close to zero in this area, and therefore optimal operation of the sensor, without the need to consume all the oxygen in the solution (which is difficult to achieve and in any case more time consuming and more expensive). The removal of oxygen is paired with the formation of water only, with no formation of unwanted compounds like H₂O₂.

Moreover, it is advantageous to avoid the use of toxic deoxygenation chemicals. Thus, in one aspect, the present invention relates to the use of a device comprising :
∘ A porous electrode ;
∘ In contact with said porous electrode, an electrically insulating porous layer;
to reduce or remove the oxygen dissolved from a solution in contact with a working electrode by contacting said electrically insulating porous layer of said device with said working electrode.

In another aspect, the present invention relates to a process of reducing or removing oxygen from a solution in contact with a working electrode comprising the steps of:
a) Contacting the electrically insulating porous layer a device comprising:
   ∘ A porous electrode ; and
   ∘ In contact with said porous electrode, an electrically insulating porous layer;
   with said working electrode; and
b) Applying a potential to the porous electrode.

In another aspect, the present invention relates to the use of a device comprising :
∘ A porous electrode ;
∘ In contact with said porous electrode, an electrically insulating porous layer; and
∘ In contact with said electrically insulating porous layer, a working electrode; to detect and/or quantify an analyte dissolved in a solution further comprising oxygen.

In another aspect, the present invention relates to a process of detecting and/or quantifying an analyte dissolved in a solution further comprising oxygen, comprising the steps of:
a) Contacting the electrically insulating porous layer a device comprising:
   ∘ A porous electrode ;
   ∘ In contact with said porous electrode, an electrically insulating porous layer; and
   ∘ In contact with said electrically insulating porous layer, a working electrode;
b) When performing step c), and optionally before performing step c), applying a potential to the porous electrode;
c) Detecting and/or quantifying said analyte using said working electrode.

In particular, and further to the working electrode defined above (also referred as the first working electrode), the porous electrode is a second working electrode. Indeed, the porous electrode is a working electrode enabling the reduction of dissolved oxygen into water, whereas the first working electrode is a working electrode as well known by the skilled person in the art, in particular in the context of electroanalysis of a given analyte, within a electrochemical sensor, of electrochemical conversion, for example NAD(P)+ regeneration, or as a biocathode, for example for the nitrate reduction.

In a particular embodiment, the porous electrode is constituted of or comprises a metal and/or carbon.

In a particular embodiment, the porous electrode is constituted of or comprises at least one porous, electrically conductive material, in particular chosen from metal grids, metal meshes, metal nets, metal lattices, carbon papers, more particularly graphitized or carbonized carbon fiber papers, metallized carbon paper, carbon fiber nonwovens, woven carbon fiber fabrics, carbon or graphite felts, beds of carbon or graphite particles, or combinations thereof.

It is noted that the porous electrode may be constituted of or comprised a plurality of the above-mentioned materials, for example a plurality of metal grids, more particularly 2 or 3 stacked metal, for example Pt, grids.

In a more particular embodiment, the metal is chosen from the group comprising platinum, stainless steel, palladium, rhodium, ruthenium, gold or combinations thereof.

In a more particular embodiment, the metal of the porous electrode is constituted of or comprises a platinum or stainless steel grid or mesh.

In a more particular embodiment, the carbon is chosen from carbon papers.

In a more particular embodiment, the carbon is chosen from the group comprising metal modified carbon papers, in particular platinum modified carbon papers, more particularly platinum coated carbon papers.

As mentioned above, the metal the porous electrode may be constituted of or comprise a plurality of modified carbon papers, in particular platinum modified carbon papers, more particularly platinum coated carbon papers, for example 2 or 3.

In a particular embodiment, the thickness of the porous electrode is comprised from about 1 to about 1000 µm, in particular from about 10 to about 200 µm, more particularly from about 50 to about 100 µm.

In a particular embodiment, the size of the pores of the porous electrode is comprised from 1 to 500 µm in particular from about 10 to about 250 µm.

In a more particular embodiment, the size of the pores of the porous electrode is comprised from 1 to 500 µm in particular from about 10 to about 250 µm, and the thickness of the porous electrode is comprised from 2 to 10 times the size of the pores of the porous electrode, notably from 2 to 5 times.

In a particular embodiment, the electrically insulating porous layer is constituted of or comprises a polymer, or an inorganic material.

In a particular embodiment, the electrically insulating porous layer is constituted of or comprises a membrane, grid, mesh, woven fiber fabric, fiber nonwoven, in particular a polymer membrane or a polymer grid, more particularly a polyamide grid.

Said membrane is for example a membrane for filtration, as well known by the skilled person in the art.

In a more particular embodiment, the polymer is chosen from polyamide, polyimide, polyester, polyethylene, polytetrafluoroethylene.

In another more particular embodiment, the inorganic material is chosen from glass, in particular sintered glass, and ceramics, in particular sintered ceramics, said ceramics being notably made from clay, quartz, alumina, feldspar or their mixtures.

In a particular embodiment, the thickness of the electrically insulating porous layer is comprised from about 10 nm to about 500 µm, in particular from about 1 µm to about 200 µm, more particularly from about 10 to about 100 µm, even more particularly from about 50 to about 100 µm.

In a particular embodiment, the size of the pores of the electrically insulating porous layer is comprised from 0.002 to 500 µm, in particular from about 0.1 µm to about 100 µm.

In a more particular embodiment, the size of the pores of the electrically insulating porous layer is comprised from 0.002 to 500 µm, in particular from about 0.1 µm to about 100 µm, and the thickness of the electrically insulating porous layer is comprised from 2 to 10 times the size of the pores of the electrically insulating porous layer, notably from 2 to 5 times.

The applied potential is a potential that enables the conversion of dioxygen into water thanks to the porous electrode ("second working electrode"). This applied potential is easily determined by the skilled person in the art, for example by varying said potential and note the values that enable a good conversion of dioxygen into water.

In a particular embodiment, the potential applied during step b) is a fixed potential.

In a particular embodiment, the potential applied during step b) is a potential comprised from 2 to -1 V, in particular vs. Ag/AgCl, more particularly of about 0.6 to about - 0.8 V vs. Ag/AgCl.

For example, the potential may be of about -0.7 V vs Ag/AgCl for a stainless steel based porous electrode ("second working electrode"), or of about -0.4 to about -0.3 V vs Ag/AgCl for a platinum based porous electrode ("second working electrode").

In a particular embodiment, the analyte is chosen from:
- organic compounds, in particular herbicides, more particularly paraquat; insecticides, more particularly imidaclopride; organic solvents, more particularly dimethylsulfoxyde; glucose; glutathion disulfide; trinitrotoluene; NAD(P)+ ;
- inorganic compounds, in particular hydrogen peroxide; monochloramine; inorganic ions, more particularly nitrate, nitrite, chromate, perchlorate, bromate, or metal ions, the metal being for example Cu, Cd, Pb, Hg;
- gas, with the proviso the gas is not dioxygen, in particular H₂, CO₂, SO₂.

In a particular embodiment, the working electrode is a porous working electrode.

In another aspect, the present invention concerns a device comprising:
∘ A porous electrode for reducing into water oxygen dissolved in a solution;
∘ In contact with said porous electrode, an electrically insulating porous layer; and
∘ In contact with said electrically insulating porous layer, a working electrode.

All the particular embodiments mentioned above are applicable here, alone or in combination.

In another aspect, the present invention concerns a process of preparation of a device as define above, comprising:
- A step of contacting said electrode with said electrically insulating porous layer; and
- a step of contacting said electrically insulating porous layer with said working electrode.

The two steps can be performed in any order, in particular in the order as mentioned above.

In particular, the two contacting steps, independently from each other, can be performed by any mechanical methods known by the skilled person in the art, for example by mechanical pressing, gluing or using an adhesive, preferably on the periphery of the device to obtain.

### Definitions

The following terms and expressions contained herein are defined as follows:
As used in this description, the term "about" refers to a range of values of ± 10% of a specific value. For example, the expression "approximately 100 µm" includes values of 100 µm ± 10%, i.e. values from 90 µm to 110 µm.

As used herein, a range of values in the form "x-y" or "x to y", or "x through y", include integers x, y, and the integers therebetween. For example, the phrases "1-6", or "1 to 6" or "1 through 6" are intended to include the integers 1, 2, 3, 4, 5, and 6. Preferred embodiments include each individual integer in the range, as well as any subcombination of integers. For example, preferred integers for "1-6" can include 1, 2, 3, 4, 5, 6, 1-2, 1-3, 1-4, 1-5, 2-3, 2-4, 2-5, 2-6, etc.

By "porous" is in particular meant a material containing pores, arranged in such a way that a liquid, in particular an aqueous solution, is able to circulate through the material.

By "pore" is in particular meant a cavity in a material, said cavity being for example located within the material, or in contact with one or more of the surfaces of the material.

### FIGURES

**Figure 1** is a scheme of the open, stirred four-electrode electrochemical cell used for the oxygen removal according to example 1.
**Figure 2** presents in relation with example 1 the cyclic voltammogram (CV) concerning: (A) a comparison of oxygen reduction on WE1 with applying WE2 (SS) at -0.7 V and under N₂ at scan rate 100 mV s⁻¹; (B) oxygen reduction at scan rate 10 mV s⁻¹ on WE1 applying WE2 (Pt) at -0.4 V.
**Figure 3** presents in relation with example 2 the cyclic voltammogram (CV) of 20 µM paraquat in 0.07 M NaNO₃ at silica thin film modified GCE (A) "filter" curve by applying - 0.5V at WE 2 for O₂ reduction, "under air" curve without applying potential at WE 2 in the presence of oxygen, (B) "filter" curve by continuous N₂ purging to liberate O₂ from solution, "under N₂" curve by applying -0.5 V vs Ag/AgCl at WE2 to reduce oxygen. Scan rate = 20 mv s⁻¹.
**Figure 4** presents in relation with example 3 the scanning chronopotentiometric analysis performed with a screen printed carbon electrode modified with mercury in 0.1 M NaNO₃ at pH 3 solution in the presence of 4x10⁻⁸ mol/L Cd²⁺. (A) Oxygen is present in solution. (B) Oxygen is removed by the filter made of platinum grids (200 µm thickness) on a Nylon grid (50 µm thickness).
**Figure 5** presents in relation with example 4 the cyclic voltammograms of oxygen reduction on GCE in 100 mM KCl (A) applying Pt filter and under N₂, (B) Comparison between with/without using filter. Scan rate at 5 mV s⁻¹.
**Figure 6** presents in relation with example 5 the CV of oxygen reduction under convection on GCE in 100 mM KCl. Comparison between using filter ("Filter on") and under N₂. Scan rate at 5 mV s⁻¹.
**Figure 7** presents (A) the catalytic current measured from chronoamperometry versus NAD⁺ concentration recorded on [Cp^{∗}Rh(bpy)Cl]⁺ functionalized bucky paper electrode in 50 mM PBS (pH 6.5) at an applied potential of -0.78 V vs Ag/AgCl. (B) a comparison the amperometric response by using oxygen filter and under N₂ atmosphere with 1 mM NAD⁺ addition. (C) the UV-vis spectroscopy of NADH formed after 20 min by adding 4 mM of NAD⁺ in the chonoamperometry experiment. (D) the Faraday efficiency of NADH regeneration under air, nitrogen, and applying oxygen filter.

### EXAMPLES

### Example 1: An electrochemistry cell according to the invention for the oxygen removal

### Materials

Stainless steel (SS) and Platinum (Pt) grid were purchased from Goodfellow SARL with purity of 99.9 %. Membrane filters (DVPP), glassy carbon (GCE) electrode were bought from Merck Milipore and Sigradur HTWHochtemperatur-Werkstoffe, Germany, respectively.

### Cell

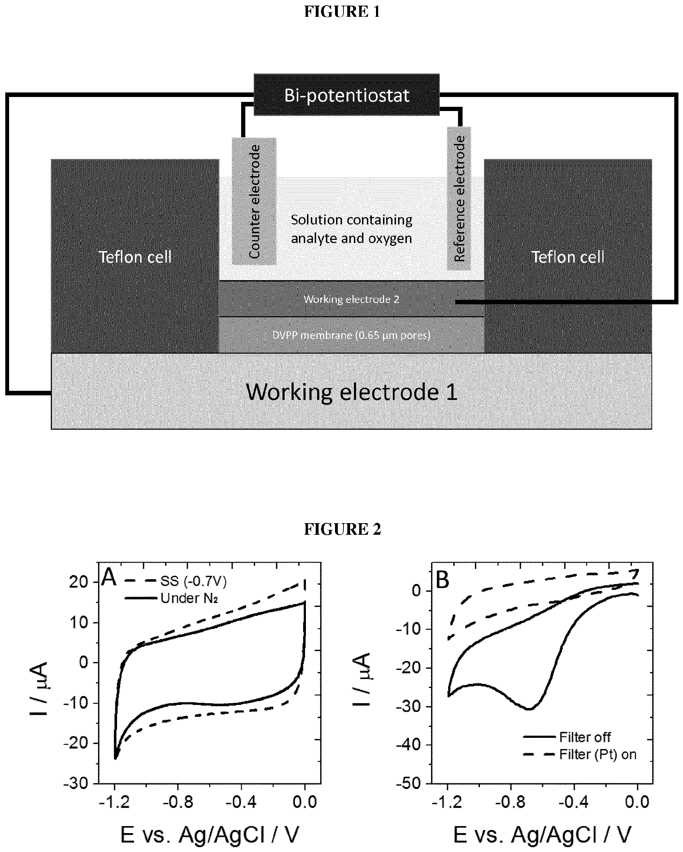

The electrochemistry cell was composed of four electrodes and fabricated from Teflon material with cylindered form as depicted in **Figure 1**. The WE1 and WE2 were separated by a DVPP membrane filter with diameter of 0.5 cm and pore size at 0.65 µm. For usage as WE1, glassy carbon was firstly wet-polished by SiC grinding paper (≠4000, Struers, Denmark) for 1 min, then cleaned with ethanol and distilled water under ultrasonic condition. SS or Pt grid was used as WE2 material, Ag/AgCl as reference electrode and a steel auxiliary bar as counter electrode. The connection of WE2 was done via Pt wire, or copper tape for WE1. O₂ removal was controlled by scanning WE1 from 0 V to -1.2 V versus (*vs*.) Ag/AgCl in the absence and presence of WE2. Prior to electrochemical tests, the applied potential at WE2 was chosen depending on the O₂ reduction peak observed on WE2 by performing the experiment with three electrodes, including working electrode (SS or Pt grid), reference electrode (Ag/AgCl) and counter electrode (steel bar). A 10 mL aqueous solution of KCl (0.1 M) was utilized as supporting electrolyte. The cyclic voltammogram (CV) was recorded on a Palm Sens 3 potentiostat at a scan rate of 100 mV s⁻¹. To repeat the performance of O₂ elimination at WE2, at each cycle the electrolyte was stirred in 30 min with a constant magnetic stirring of 600 rpm using 728 Stirrer (Ω Metrohm) for recovery of dissolved O₂ into solution from the air. The comparison experiment was done under nitrogen. Purging the solution with N₂ gas was performed at least 15 min before running experiment to saturate electrolyte and kept during the test.

### Results

The oxygen removal was investigated by scanning WE1 (Glassy carbon) in the absence or presence of SS as WE2 at fixed potential. Without applying WE2, a broad oxygen reduction in the range -0.4 V -> -0.8 V was observed on WE1. However, once the second electrode was polarized at potential of -0.7 V, the reduction wave on WE1 was very straight with complete disappearance of oxygen peak. The experiment was repeated by stirring solution in 30 min and done again the polarization steps. The similar behavior was repeatable from the first cycle to third cycles and fifth cycle. The obtained results confirmed strongly that the oxygen was nearly removed totally at surface of WE2 and prevented from reaching WE1. The comparison experiment was performed under nitrogen atmosphere. The oxygen consumption at WE2 was even better than by nitrogen saturation where a small peak with very low current was observed around -0.5 V on **Figure 2A**.

The oxygen removal efficiency at WE2 using the designed electrochemistry cell was also proved by replacing SS by another material like Pt. The acquired electrochemical feature on CV waves in both case of using and without WE2 were similar to the above results for SS. Regarding on **Figure 2B**, a sharp and strong peak was noticed from -0.6 V to -0.8 V, which indicated the presence of dissolved O₂ in electrolyte solution under air. On the contrary, no reduction peak at the same potential range in case of polarizing Pt at -0.4 V. In conclusion, the concept of O₂ elimination at second electrode was successfully carried out, and the applied potential value depended on the material using as WE2.

In sensor application, the presence of hydrogen peroxide can create some negative effects because of its high oxidation property which often used as oxidant. Therefore, the oxygen removal without H₂O₂ production is also an important factor to evaluate the efficiency of the designed cell. The testing experiments were performed by scanning WE1 in the range 1.0 V -> -1.2 V under applying of SS (WE2) at -0.7 V and adding H₂O₂ concentration from 0 mM to 1.6 mM or 3.2 mM. The addition of H₂O₂ increased clearly the anodic response near 0.6 V, which came from the H₂O₂ oxidation. There was no peak observed in this area on the curve obtained with no H₂O₂ addition, proving that the deoxygenation on WE2 did not generated other side products like H₂O₂.

### Example 2: Electrochemical sensing of paraquat

Paraquat (1,1-dimethyl-4,4 bipyridinium dichloride) is a cationic redox chemical compound of the viologen family, highly soluble in water. Paraquat is used as herbicide to control broad leaf weeds in agricultural practices since the 1960s. It is used worldwide in more than 100 countries but is banned in European Union. It causes severe toxicity to living organisms by damaging the lungs, kidneys, liver and heart. It is also reported to cause Parkinson's disease. Therefore, it is necessary to detect this compound at small concentration in aqueous medium. At low concentration, paraquat is detected by electrochemical method via running the cycle voltammograms. There is the appearance of two successive one electron reactions at -0.7V and -1.025V on reduction curve and then oxidize back to the relative potential values. However, these processes cannot be observed in case the presence of dissolved oxygen and the detection efficiency depends on the O₂ removal ability. For electrochemical sensing of paraquat, study of the first redox reaction is enough to provide sufficient quantification of paraquat in the aqueous media. However, the first single electron reduction of paraquat is at - 0.7 V at GCE which is almost at the same potential where reduction of oxygen takes place.

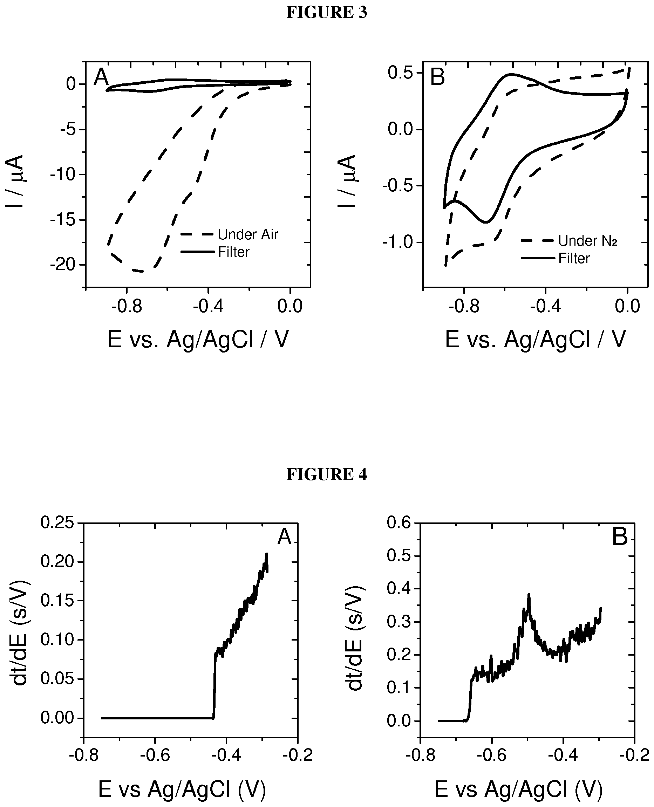

As a result, the signal of paraquat is suppressed (**Figure 3**, "under air" curve) by oxygen reduction.

The paraquat at concentration of 20 µM was detected under air on a silica thin film modified GCE (WE1) using designed electrochemistry cell with the polarization of Pt as WE2 at fixed potential of -0.5 V, as described in Example 1. Sodium nitrate (0.07 M) was used as electrolyte.

### Results

The attained result was compared with the one done under N₂ saturation or without applying WE2. The CV was also recorded on a Palm Sens 3 potentiostat at a scan rate of 20 mV s⁻¹. All potentials in this study were quoted with respect to the Ag/AgCl.

As described in example 1, WE1 was used for paraquat analysis and WE2 for the continuous reduction of oxygen by applying -0.5V potential during the experiment. In **Figure 3A**, the paraquat signal was suppressed due to the presence of oxygen but after the reduction of O₂ at WE2 the paraquat signal was greater. In **Figure 3B** the paraquat signal when recorded by applying potential at WE2 showed even better response as compared to N₂ purging. This result was linked to the better oxygen consumption by applying WE2 in comparison with N₂ saturation, proving the high efficiency of the uses, processes and devices of the invention for O₂ removal in sensor application.

### Conclusion

In this example, two kinds of WE2 materials, SS and Pt, were tested and the similar results were obtained for both. In fact, the polarization of SS at -0.7 V vs Ag/AgCl or -0.4 V vs Ag/AgCl for Pt could eliminate oxygen reduction peaks from -0.4 V to -0.8 V vs Ag/AgCl on the GCE as WE1. The experimental results were repeatable at least to five cycles. Importantly, the experiment about the electrochemical oxidation of hydrogen peroxide confirmed that there was no H₂O₂ generation during the deoxygenation at WE2. Additionally, the effect of O₂ removal was clearly proved in case of using the designed cell to detect an herbicide compound, paraquat. Paraquat at concentration of 20 µM was well detected under air on silica thin film modified GCE (WE1) by applying a fixed potential at -0.5 V on Pt (WE2). The obtained results showed that the O₂ elimination at WE2 was better than under N₂ atmosphere.

### Example 3: Electrochemical oxygen filter according to the invention for the detection of cadmium

Two experiments are reported here to illustrate the interest of O₂ filter for the detection of cadmium. **Figure 4A** reports the tentative of electrochemical detection of cadmium in solution by scanning chronopotentiometry in the presence of oxygen. In this experiment, the sensor was introduced in solution and a potential of -0.75 V (vs. Ag/AgCl) was applied to the electrode for 45 s before scanning chronopotentiometry. The result of this experiment is a graph that link dt/dE versus the electrode potential. The introduction of 4x10⁻⁸ mol/L cadmium modifies slightly the profile of the curve, with a sharp increase of the signal between -0.4 and -0.45 V, but no peak related to cadmium was observed. **Figure 4B** reports a similar experiment performed with the oxygen filter made of Pt grids and a Nylon grid according to the invention. The experiment in the absence of cadmium shows that the sharp increase of signal is observed at -0.65 V, a potential lower than the one measured in the absence of oxygen filtration. This is due to the low concentration of oxygen when the oxygen filter is used. This low concentration of oxygen allows to detect 4x10⁻⁸ mol/L cadmium at -0.5 V. Moreover, the peak intensity of this signal at -0.5 V is increasing linearly with the concentration of cadmium.

### Example 4: NAD⁺ detection and NAD(P)H regeneration using a platinum grid based oxygen filter according to the invention

Coenzyme/cofactor, β-nicotinamide adenine dinucleotide NAD(P)H, has been widely used in biocatalysts as a hydride source for the synthesis of chiral compounds. During the enzymatic reduction process, NAD(P)H played the role of a reductant that is *in situ* oxidized to NAD(P)⁺, and the conversion occurs stoichiometrically. Regarding on its high cost, the development of regeneration approaches for continuous recycling of this cofactor is very crucial for practical application. The NAD(P)H regeneration requests transfer of two electrons and a proton to NAD(P)⁺ from the hydride donors. Among the known methods, the electrochemical NAD(P)H regeneration strategy presents some remarkable advantages relevant to its potentially low cost and without reducing agent addition which is important for the product isolation. However, the interfering oxygen removal is essential to expel the O₂ reduction process occurred at the same catalytic potential range on carbon electrodes, which hinders the NAD(P)H regeneration.

Although showing a good efficiency for O₂ removal, the oxygen elimination methods of the prior art [e.g. physical (bubbling nitrogen/argon flow in the solution), chemical (adding oxygen reducing agent such as ascorbic acid (C₆H₈O₆)] kill total oxygen species in reaction area and cannot be applied for the electroenzymatic synthesis demanding the O₂ supply.

### Material

Platinum grid (99.9 %) and polyamide - Nylon grid (39 µm wire diameter, 50 µm nominal space) were purchased from Goodfellow, England. Multi-walled carbon nanotubes (MWCNT, > 95%, Φ 6-9nm, L 5µm).

### Removal of oxygen from aqueous medium

The oxygen removal from aqueous medium was carried out in an open cylindrical electrochemical reactor. This system included four electrodes with an Ag/AgCl/1 M KCl (purchased from Metrohm, Switzerland) as reference electrode, platinum grid (0.1 mm wire diameter, 0.4 mm nominal space) as counter electrode, the glassy carbon (GCE) as working electrode 1 (WE1, S = 16.61 mm²) and platinum grid (0.04 mm wire diameter, 0.12 mm nominal space) as working electrode 2 (WE2, S = 16.61 mm²). The effect of WE2 layer numbers to O₂ removal efficiency was simultaneously examined. Between WE1 and WE2 was separated by a porous Nylon grid which allowing oxygen easily transfer from solution to surface of WE1. The connection of both WE1 and WE2 was done via Pt wires. Oxygen presented in 10 mL of supporting electrolyte (KCl 100 mM) under air was eliminated by applying a constant potential (-0.4 V vs. Ag/AgCl) at WE2. The oxygen absence was recorded on WE1 by running cyclic voltammograms using Autolab (PGSTAT 100). A similar experiment was also performed by disconnecting of WE2 to see the O₂ presence on WE1. In addition, the O₂ disappeared signal in the case of WE2 connection was confirmed by running experiment in a closed reactor under N₂ bubbling.

### NAD(P)H regeneration

A Bucky paper electrode immobilized with [Cp^{∗}Rh(bpy)Cl]⁺ (BP-Bpy-Rh) was prepared following the protocol published by Zhang et al. ("Electrocatalytic biosynthesis using a bucky paper functionalized by [Cp∗Rh(bpy)Cl]+ and a renewable enzymatic layer," ChemCatChem, 2018). This functionalized electrode was used for NADH or NADPH regeneration by observation of the catalytic peak on BP-Bpy-Rh during NAD⁺/NADP⁺ addition. This reaction is very sensitive with O₂ presence, so it is essential to avoid completely the oxygen presence. The oxygen removal was also carried out by applying a constant potential (-0.4 V vs. Ag/AgCl) at WE2 as explained above. However, in this case, replacing GCE by BP-Bpy-Rh as WE1 where catalytic response was evaluated by running cyclic voltammetry from -0.4 V to -0.9 V vs. Ag/AgCl at scan rate of 5 mV s⁻¹. Some comparison experiments were done to prove : (1) The O₂ removal capacity of WE2 filter by repeating experiment under N₂ bubbling ; (2) The catalytic role of [Cp^{∗}Rh(bpy)Cl]⁺ for NAD(P)H regeneration by doing experiment with bare electrode (bucky paper) in N₂ medium; (3) The sensitivity with oxygen during the reduction of NAD(P)⁺ by performing experiment under air without WE2 connection.

Towards the bioconversion of D-fructose to D-sorbitol, a compact cell was designed, including different layers. Firstly, D-sorbitol dehydrogenase (DSDH) in silica gel which called DSDH sol was deposited on microfiber filter layer. The DSDH sol was synthesized by stirring overnight a mixture of 0.13 g GPS, 0.18 g TEOS with 0.5 mL water and 0.625 mL 0.01 M HCl. The day after, the sol was diluted three times before mixing 40 µL this aliquot with 20 µL of PEI (20%), 20 µL of H₂O and 30 µL of DSDH stock solution. Then, it is necessary to dry this DSDH gel layer at 4 °C overnight. After that, it was put on the top of WE2, following to Nylon separator and BP-Bpy-Rh electrode as WE1. Platinum grid (0.1 mm wire diameter, 0.4 mm nominal space) and Ag/AgCl/1 M KCl were still used as counter electrode and reference electrode, respectively. Prior of experiment, 1 mM NADH was mixed in 10 mL of buffer phosphate solution (PBS, 50 mM pH = 6.5). The transformation of D-fructose to D-sorbitol on the surface of DSDH gel layer consumed NADH which was regenerated at WE1. In this system, two layers of platinum grid (0.04 mm wire diameter, 0.12 mm nominal space) was used as WE2 for oxygen filter during the NADH regeneration.

### Results

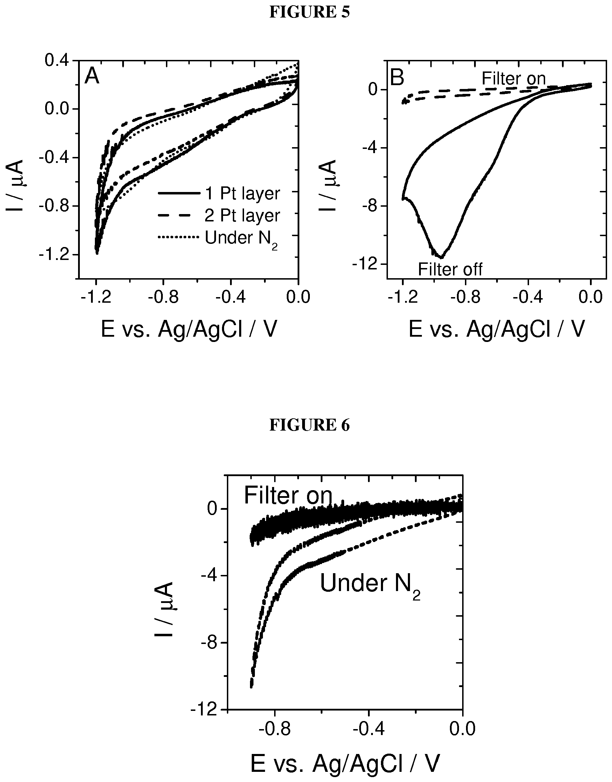

The oxygen removal experiment was carried out in a four-electrode system with platinum grid (0.1 mm wire diameter, 0.4 mm nominal space) as counter electrode, Ag/AgCl/1 M KCl as reference electrode, the glassy carbon (GCE) as working electrode 1 (WE1, S = 16.61 mm²) and platinum grid (0.04 mm wire diameter, 0.12 mm nominal space) as working electrode 2 (WE2, S = 16.61 mm²). The oxygen presence was detected on WE1, and oxygen was consumed at WE2. **Figure 5A** displayed the complete oxygen elimination by applying a constant reduction potential (-0.4 V) at WE2 with one Pt layer or two Pt layers. This elimination was realized under N₂ bubbling with the absence of O₂ peak on WE1 (GCE). Without applying WE2 in the case of filter off in **Figure 5B**, the O₂ reduction was observed obviously with the current value of 11 µA at -0.95 V. This consequence proved that using the electrochemical cell according to the invention as WE2 could avoid interference oxygen molecules coming to the WE1. This is very important to proceed the experiments sensitive with oxygen like NAD(P)H regeneration.

For NAD(P)H regeneration, [Cp^{∗}Rh(bpy)Cl]⁺ was chosen as an efficient non-enzymatic catalyst. The immobilization of [Cp^{∗}Rh(bpy)Cl]⁺ on the surface of bucky paper (BP) was achieved via electro-grafting and complexation steps. By applying a negative potential on the BP-Bpy-Rh electrode, the Rh(III) on the surface of electrode was reduced to Rh(I). The catalytic response of [Cp^{∗}Rh(bpy)Cl]⁺ was noticed around -0.65V by running CV without adding NAD⁺ in 10 mL of PBS (50 mM, pH = 6.5) from -0.4 V to -0.9 V vs. Ag/AgCl. This experiment was performed in above four-electrode cell. Platinum grid (0.1 mm wire diameter, 0.4 mm nominal space) and, Ag/AgCl/1 M KCl were still used as counter electrode and reference electrode, respectively. Applying reduction potential (-0.4 V) at WE2 to remove oxygen approaching to WE1 where the catalytic response of [Cp^{∗}Rh(bpy)Cl]⁺ was observed. The peak at -0.65 V was also obtained when the experiment was repeated under N₂ medium. The absence of [Cp^{∗}Rh(bpy)Cl]⁺ immobilization on the surface of BP electrode, there was no peak presented on WE1. The oxygen presence by disconnection WE2 covered the catalytic peak by a large peak in the range from -0.4 V to -0.7 V.

In conclusion, the oxygen presence could be detected while performing NAD⁺ reduction reaction, and NADH regeneration could be performed under air medium by using the cell of the invention for O₂ removal at WE2.

### Conclusion

Applying a reduction potential (-0.4 vs. Ag/AgCl) at WE2 which was composed for example by two layers of platinum grid (0.04 mm wire diameter, 0.12 mm nominal space) could remove totally oxygen and no oxygen was detected at WE1 (glassy carbon electrode). Then, NAD⁺ reduction reaction at the surface of BP-Bpy-Rh electrode was chosen to detect the oxygen presence because of its sensitivity to O₂. Without applying WE2, a board peak in the range from -0.4 V to -0.7 V corresponding to O₂ presented in solution, hided the catalytic response of rhodium complex. The oxygen removal by filter made the cathodic peak appear around -0.65V without NAD⁺ addition, and current value increased from 35 µA to 60 µA by adding 0.25 mM of NAD⁺. The result was repeated by running experiment under N₂. This phenomenon also happened in the case replacing NAD⁺ by NADP⁺ and the catalytic current reached to the saturated value at 100 µA. Therefore, it could be concluded that NAD(P)+ could be detected under air medium by applying our oxygen filter, and NAD⁺ reduction reaction is sensitive reaction to O₂.

### Example 5: NADH regeneration on Rh-based electrode using a platinum modified carbon paper based oxygen filter of the invention

### Materials

Carbon paper (CP, thickness 80 µm, CeTech GDS090) were bought from FuelCellStore. Sodium hexachloro-platinate (IV) hexahydrate (Na₂PtCl₆.6H₂O, 98%), multi-walled carbon nanotubes (MWCNT, > 95%, Φ 6-9nm, L 5µm, were obtained from Sigma-Aldrich. Nylon grid (39 µm wire diameter, 50 µm nominal space, Goodfellow), platinum grid (0.04 mm wire diameter, 0.12 mm nominal space, Goodfellow), were used without any purification.

### Electrodeposition of Pt on carbon paper electrode

The commercial CP was firstly cleaned in an ultrasonic bath with the mixture of ethanol/H₂O0 to eliminate any impurity resulting from the industrial manufacturing process. In addition, the cleaning step helped to reduce the hydrophobicity of CP, which was necessary for Pt deposition in next step occurred in aqueous medium. This pretreated carbon felt was denoted as raw CP. The Pt was electrodeposited on the surface of CP by the method of CV running 30 cycles from 0 to -1 V vs. Ag/AgCl at a scan rate of 20 mV s⁻¹ in Na₂PtCl₆ solution under nitrogen atmosphere to avoid the oxygen reduction reaction (ORR). The process was recorded on a Autolab (PGSTAT 100) using a three-electrode system with the CP as working electrode, an Ag/AgCl/1 M KCl (purchased from Metrohm, Switzerland) as reference electrode and platinum grid as counter electrode. After the electrodeposition step, the sample was rinsed and dried at 70 °C. The as-prepared sample was labeled as « Carbon paper + Pt » or Pt@CP.

### Platinum modified carbon paper for oxygen filter under convection

The oxygen filter system was an electrochemical Teflon cylinder cell with round working area of 16.61 mm². It was compacted of Pt@CP (working electrode 2, WE2), Nylon separator, glassy carbon (working electrode 1, WE1). An Ag/AgCl/1 M KCl and platinum grid as reference electrode and counter electrode, respectively were played in the solution of KCl 100 mM. In this system, WE2 played the role of oxygen filter which prevented O₂ molecules passing from solution to WE1. The experiment was perform under convection by stirring at a rate of 800 rpm. The oxygen presence at the surface of WE1 was detected by running experiment from 0 V to -0.9 V vs. Ag/AgCl, and applying a reduction potential at WE2. To evaluate the efficiency of oxygen filter, different parameters were evaluated, including : various amount of Pt deposited on carbon paper, potential valued applied on WE2, number of Pt@CP. The O₂ removal by using the filter was confirmed by repeating the experiment under nitrogen bubbling.

### Applying oxygen filter for NADH regeneration under convection in the air condition

The oxygen filter was used to regenerate NADH. To perform this experiment, firstly, a Bucky paper electrode (BP) was prepared from dispersion of MWCNT in 50 mL ethanol by ultrasonication for 5 h. Then the rhodium catalyst ([Cp^{∗}Rh(bpy)Cl]⁺) was immobilized on BP. The NAD⁺ transformation was carried out via the chronoamperometry experiment with applied potential of -0.78 V. Different amount of NAD⁺ was added gradually to the buffer phosphate solution (PBS, 50 mM, pH = 6.5) to measure catalytic current versus NAD⁺ concentration recorded on [Cp^{∗}Rh(bpy)Cl]⁺ functionalized Bucky paper electrode (BP-Bpy-Rh). In this case, WE1 was BP-Bpy-Rh, WE2 was Pt@CP and reference electrode as well as counter electrode were kept unchanged. The formed NADH concentration was identified by absorbance at 340 nm by UV-Vis spectroscopy. UV-Vis spectra have been recorded on a Cary 60 Scan UV-Vis spectrophotometer. The NADH regeneration was repeated under nitrogen and air atmosphere (without applying WE2) to compare the faraday efficiency of the transformation.

### Pt@CP for oxygen filter under convection

The oxygen removal was performed in a four-electrode cell, including platinum grid (counter electrode), Ag/AgCl/1 M KCl (reference electrode), glassy carbon (GCE, working electrode 1), and Pt@CP or Pt grid (working electrode 2). Applying a reduction potential (-0.4 V) at WE2 could eliminate completely interference oxygen. The O₂ absence on WE1 was confirmed by running CV from 0 V to -0.9 V at a scan rate of 5 mV s⁻¹ in 100 mM KCl under convection. By disconnecting WE2, oxygen presented in the solution under air passed easily to WE2 as well as nylon separator and came to the surface of GCE. Therefore, a huge reduction current was observed around -0.6 V to -0.8 V vs. Ag/AgCl which was assigned for ORR.

To prove the efficiency of the filter for oxygen removal, the experiment was repeated under N₂ bubbling in **Figure 6**. It was noticed that the interference oxygen molecules were totally eliminated at the filter, while it was seemly difficult to perform even by running experiment under nitrogen atmosphere. When potential was applied on WE2, all of oxygen molecular at electrode surface was immediately reduced and its concentration at the surface of filter was nearly to zero. Consequently, O₂ could not pass through the filter and come to WE1.

### Pt@CP) filter for NADH regeneration under convection

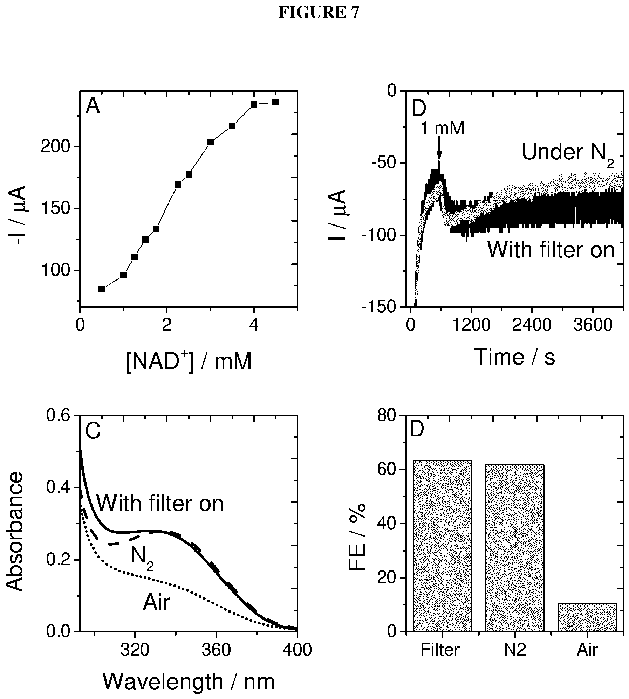

To carry out this experiment, Bucky paper functionalized by rhodium complexe (BP-Bpy-Rh) with 35.3 µm of thickness was placed at WE1 in which happened the NAD⁺ reduction. Two Pt@CP layers, Ag/AgCl/1 M KCl and platinum grid were kept unchanged as WE2, reference electrode and counter electrode, respectively. The gradual NAD⁺ concentrations were added in 10 mL solution of PBS (50 mM, pH = 6.5) during the chronoamperometry experiment with applied potential of -0.78 V at BP-Bpy-Rh electrode. **Figure 7A** pointed out that the catalytic current increased nearly linear with the NAD⁺ rise and attained to saturated value at 4 mM. As shown in **Figure 7B**, the catalytic current to 1 mM of NAD⁺ addition was kept at the same level for both cases of using filter and under N₂ atmosphere. It was noticed that the catalytic current by using oxygen filter was more stable that N₂ bubbling, which confirmed the better performance for O₂ removal. The produced NADH was calculated by measuring the absorbance at 340 nm using UV-Vis spectroscopy (**Figure 7C**). The NADH production was done in three mediums like using filter, under N₂ atmosphere, and under air that meant disconnected with WE2. The formed NADH was measured after 20 min adding of 4 mM NAD⁺ in PBS solution. The O₂ presence at the surface of WE1 in case of without using filter, hinder significantly the NADH regeneration. In consequence, a few amount of NADH was detected while NAD⁺ reduction occurred efficiently in the O₂ absence medium by using filter or N₂ bubbling. From calculated NADH concentrations, the faradaic efficiency (FE) values were obtained at 63.4 %, 61.7 % and 10.6 % for O₂ filter, under N₂ and under air, respectively (**Figure 7D**). In fact, the usage of oxygen filter could improve 52.8 % of FE and brought back a stable catalytic current, proving the excellent performance of designed filter for bio-electrochemical applications.

### Conclusions

Platinum particles were grown successfully on the surface of carbon paper electrode via electrochemical method by running CV Na₂PtCl₆ solution with 30 cycles from 0 to -1 V vs. Ag/AgCl. The Pt deposited on CP was confirm by various physical characterization such as SEM, XRD and EDX. The electrochemical performance toward the ORR was improved by Pt modification and 0.4 mg cm⁻² was chosen as the optimal Pt amount. Then the fabricated Pt@CP was used as material for oxygen filter in a four-electrode cell. Total interference oxygen was removed at filter which was compacted by two Pt@CP layers at applied potential of -0.4 V vs. Ag/AgCl. By evaluating the effect of applied potential at WE2 to the oxygen removal efficiency, it could be noticed that a suitable value could be from -0.3 V to -0.4 V. The O₂ elimination capacity of filter was proved by repeating the same experiment under nitrogen atmosphere. Interestingly, oxygen was removed better in case of using the filter of the invnetion. This brought to more stable of catalytic current during the NADH regeneration at BP-Bpy-Rh electrode. From that, a faradaic efficiency (FE) values of 63.4 % was obtained which was nearly 6 times higher than without applying filter at WE2. In conclusion, Pt@CP was an excellent material filter at WE2 to remove completely oxygen under convection, which was very useful for applications in sensing and electrocatalysis.

## Claims

1. Use of a device comprising :
∘ A porous electrode ;
∘ In contact with said porous electrode, an electrically insulating porous layer;
to reduce or remove the oxygen dissolved from a solution in contact with a working electrode by contacting said electrically insulating porous layer of said device with said working electrode.

2. A process of reducing or removing oxygen from a solution in contact with a working electrode comprising the steps of:
a) Contacting the electrically insulating porous layer a device comprising:
∘ A porous electrode ; and
∘ In contact with said porous electrode, an electrically insulating porous layer;
with said working electrode; and
b) Applying a potential to the porous electrode.

3. Use of a device comprising :
∘ A porous electrode ;
∘ In contact with said porous electrode, an electrically insulating porous layer; and
∘ In contact with said electrically insulating porous layer, a working electrode;
to detect and/or quantify an analyte dissolved in a solution further comprising oxygen.

4. A process of detecting and/or quantifying an analyte dissolved in a solution further comprising oxygen, comprising the steps of:
a) Contacting the electrically insulating porous layer a device comprising:
∘ A porous electrode ;
∘ In contact with said porous electrode, an electrically insulating porous layer; and
∘ In contact with said electrically insulating porous layer, a working electrode;
b) When performing step c), and optionally before performing step c), applying a potential to the porous electrode;
c) detecting and/or quantifying said analyte using said working electrode.

5. The use or process as defined in any one of the preceding claims, wherein the porous electrode is constituted of or comprises a metal and/or carbon.

6. The use or process as defined in any one of the preceding claims, wherein the porous electrode is constituted of or comprises a porous, electrically conductive material, in particular chosen from metal grids, metal meshes, metal nets, metal lattices, carbon papers, more particularly graphitized or carbonized carbon fiber papers, metallized carbon paper, carbon fiber nonwovens, woven carbon fiber fabrics, carbon or graphite felts, beds of carbon or graphite particles, or combinations thereof.

7. The use or process as defined in claim 5 or 6, wherein the metal is chosen from the group comprising platinum, stainless steel, palladium, rhodium, ruthenium, gold or combinations thereof.

8. The use or process as defined in any one of the preceding claims, wherein the thickness of the porous electrode is comprised from 1 to 1000 µm, in particular from 10 to 200 µm, more particularly from 50 to 100 µm.

9. The use or process as defined in any one of claims 1 to 7, wherein the size of the pores of the porous electrode is comprised from 1 to 500 µm, in particular from 10 to 250 µm.

10. The use or process as defined in any one of the preceding claims, wherein electrically insulating porous layer is constituted of or comprises a polymer, or an inorganic material.

11. The use or process as defined in any one of the preceding claims, wherein electrically insulating porous layer is constituted of or comprises a membrane, grid, mesh, woven fiber fabric, fiber nonwoven, in particular a polymer membrane or a polymer grid, more particularly a polyamide grid.

12. The use or process as defined in claim 11, wherein the polymer is chosen from polyamide, polyimide, polyester, polyethylene, polytetrafluoroethylene.

13. The use or process as defined in any one of the preceding claims, wherein the thickness of the electrically insulating porous layer is comprised from 10 nm to 500 µm, in particular from 1 µm to 200 µm, more particularly from 10 to 100 µm, even more particularly from 50 to 100 µm.

14. The use or process as defined in any one of claims 1 to 12, wherein the size of the pores of the electrically insulating porous layer is comprised from 0.002 to 500 µm, in particular from 0.1 µm to 100 µm.

15. The use or process as defined in any one of claims 3 to 14, wherein the analyte is chosen from:
- organic compounds, in particular herbicides, more particularly paraquat; insecticides, more particularly imidaclopride; organic solvents, more particularly dimethylsulfoxyde; glucose; glutathion disulfide; trinitrotoluene; NAD(P)+;
- inorganic compounds, in particular hydrogen peroxide; monochloramine; inorganic ions, more particularly nitrate, nitrite, chromate, perchlorate, bromate, or metal ions, the metal being for example Cu, Cd, Pb, Hg;
- gas, with the proviso the gas is not dioxygen, in particular H₂, CO₂, SO₂.

16. A device comprising:
∘ A porous electrode for reducing into water oxygen dissolved in a solution;
∘ In contact with said porous electrode, an electrically insulating porous layer; and
∘ In contact with said electrically insulating porous layer, a working electrode.
